# EUROPEAN PATENT APPLICATION

(11) **EP 1 859 952 A1**
(43) Date of publication of application: **28.11.2007**
(21) Application number: 06729191.4
(22) Date of filing: 15.03.2006
(51) Int. Cl.: B41M 5/26

(54) **OPTICAL INFORMATION RECORDING MEDIUM AND OPTICAL INFORMATION RECORDING METHOD**

(30) Priority: 16.03.2005 JP 2005075553
(71) Applicant: Fujifilm Corporation, Minato-ku Tokyo 106-0031 (JP)
(72) Inventor: WATANABE, Kousuke Fujifilm Corporation, Kanagawa (JP); WATANABE, Tetsuya Fujifilm Corporation, Kanagawa (JP)
(74) Representative: HOFFMANN EITLE
(86) International application number: PCT/JP2006/305170
(87) International publication number: WO 2006/098385

(57) **Abstract**

To provide an optical information recording medium allowing for high-density recording and reproduction of information by the irradiation with laser light of 440 nm or less and having excellent recording and reproducing characteristics (in particular, sensitivity and degree of modulation).

An optical recording medium comprising a substrate having thereon a recording layer capable of recording information by the irradiation with laser light of 440 nm or less, wherein the recording layer comprises at least one platinum porphyrin represented by the following formula (I): 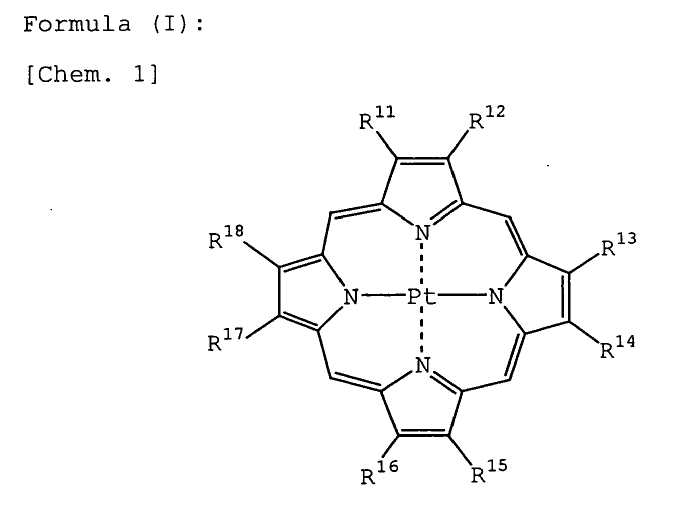 wherein _{R}11 to R¹⁸ each independently represents a hydrogen atom or a substituent.

## Description

### TECHNICAL FIELD

The present invention relates to an optical information recording medium capable of recording and reproducing information by using laser light, an information recording method, and a novel compound suitable therefor. More specifically, the present invention relates to a heat mode-type optical information recording medium suitable for the recording of information by using short wavelength laser light at a wavelength of 450 nm or less.

### BACKGROUND ART

In the related art, an optical information recording medium (optical disc) capable of only once recording information by laser light is known. This optical disc is also called CD recordable (so-called CD-R) and in a representative structure thereof, a recording layer comprising an organic dye, a light-reflecting layer comprising a metal such as gold, and a resin-made protective layer are provided in this order in a stacked layer state on a transparent disc-like substrate. The recording of information on this CD-R is performed by irradiating laser light in the near infrared region (usually laser light at a wavelength in the vicinity of 780 nm) on the CD-R. The irradiated portion of the recording layer absorbs light and causes local elevation of the temperature, as a result, physical or chemical changes (for example, production of pits) are generated and the optical properties thereof are changed, thereby recording the information. On the other hand, the reading (reproduction) of information is also performed by irradiating laser light at the same wavelength as the laser light for recording, and while detecting the difference in the reflectance between the portion changed in the optical properties (recorded portion) and the unchanged portion (unrecorded portion) of the recording layer, the information is reproduced.

Recently, a network such as the Internet and a high-vision TV have been rapidly spreading. Also, broadcasting of HDTV (High Definition Television) will start in the near future, and demands for a large capacity recording medium for inexpensively and easily recording image information are increasing. CD-R described above and DVD-R enabling high density recording by using visible laser light (630 nm to 680 nm) as a recording laser are assured of a certain position as a large capacity recording medium but cannot be said to have a recording capacity sufficiently large to cope with the requirement in the future. Accordingly, studies are being made to increase the recording density by using laser light at a still shorter wavelength than DVD-R and thereby develop an optical disc having a larger recording capacity. For example, an optical recording disc called Blu-ray system using a blue laser of 405 nm has been introduced into the market.

With respect to an optical information recording medium having a recording layer containing an organic dye, a recording and reproducing method of irradiating laser light at a wavelength of 530 nm or less toward the light-reflecting layer side from the recording layer side, thereby recording and reproducing information, has been disclosed. More specifically, there has been proposed an image recording and reproducing method of performing the recording and reproduction of information by irradiating a blue (wavelength: 400 to 430 nm, 488 nm) or blue-green (wavelength: 515 nm) laser light on an optical disc having a recording layer in which a porphyrin compound, naphthalocyanine compound or the like is used as the dye.

In the related arts, an optical recording medium recordable and reproducible by a blue laser, in which a porphyrin-based compound is used for the recording layer, has been proposed.
Patent Document 1: JP-A-11-334207
Patent Document 2: JP-A-2001-138634
Patent Document 3: JP-A-11-58957
Patent Document 4: JP-A-2001-143317
Patent Document 5: JP-A-2001-287465
Patent Document 6: JP-A-2003-272234
Patent Document 7: JP-A-2003-272259
Patent Document 8: JP-A-2004-58365
Patent Document 9: JP-A-2004-111011
Patent Document 10: JP-A-2004-160742

### DISCLOSURE OF THE INVENTION

### PROBLEMS TO BE SOLVED BY THE INVENTION

The optical recording medium using a porphyrin-based compound described in JP-A-11-334207 is recordable and reproducible by a blue laser of 488 nm, but when a blue laser of 400 to 410 nm is used, good recording and reproducing characteristics cannot be obtained. The octaethyl platinum porphyrin described in JP-A-2001-138634 has very bad solubility in an organic solvent and this makes is difficult to form a recording layer by coating and produce an inexpensive optical recording medium. Furthermore, when a blue laser of 400 to 410 nm, good recording and reproducing characteristics cannot be obtained. Therefore, it is demanded to develop an organic dye ensuring that the recording and reproduction of information by irradiation with blue laser light of 400 to 410 nm can be successfully performed.

The porphyrin-based compound specifically described in JP-A-11-334207 is a compound having a hydrogen atom, Mg, Ni, Co, Fe, Cu, Zn, Cr, In, Mn or the like in the center of the porphyrin nucleus, but it has been found that when the center metal of the porphyrin is platinum (Pt) as in the present invention, the solution absorption maximum wavelength and the film absorption maximum wavelength become short as compared with other elements (e.g., hydrogen atom, Mg, Ni, Co, Fe, Cu, Zn, Cr, In, Mn) and this allows the refractive index n and extinction coefficient k to take an appropriate value, as a result, good recording and reproducing characteristics can be obtained when using a blue laser of 400 to 410 nm.

Although the octaethyl platinum porphyrin described in JP-A-2001-138634 has very bad solubility in an organic solvent and this makes it difficult to form a recording layer by coating and produce an inexpensive optical recording medium, the solubility can be improved by using the preferred substituent in the present invention, so that coating by the use of an organic solvent can be performed and a homogenous good film can be formed.

In order to obtain good recording and reproducing characteristics, the refractive index n and extinction coefficient k of the recording layer at the oscillation wavelength of a laser of 400 to 410 nm preferably satisfy n≥2.0 and 0.01≤k≤0.4 at the same time. For this purpose, in the compound of the present invention for use in the recording layer, both the solution absorption maximum wavelength and the film absorption maximum wavelength need to be from 350 to 385 nm. This is because a compound having a solution absorption maximum wavelength exceeding 385 nm is liable to have a large molar absorption coefficient at 405 nm and can hardly satisfy the relationship of 0.01≤k≤0.4.

An object of the present invention is to provide an optical information recording medium allowing for high-density recording and reproduction of information by the irradiation with laser light of 400 to 410 nm and having excellent recording and reproducing characteristics (in particular, sensitivity and degree of modulation). Another object of the present invention is provide a dye enabling the production of such an optical information recording medium.

### MEANS TO SOLVE THE PROBLEMS

The objects of the present invention are successfully achieved by the following constitutions.

[1] An optical recording medium comprising a substrate having thereon a recording layer capable of recording information by the irradiation with laser light of 440 nm or less, wherein the recording layer comprises at least one platinum porphyrin represented by the following formula (I): Formula (I):

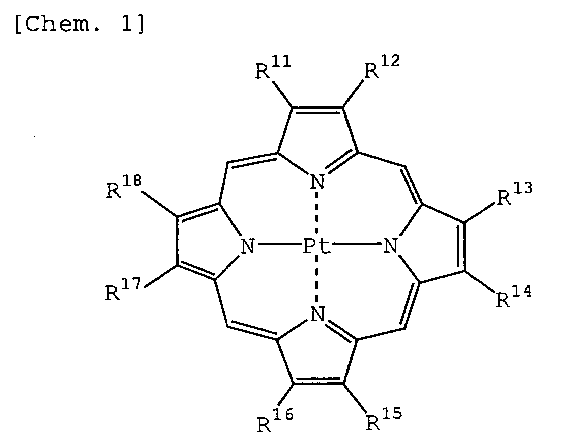

wherein R¹¹ to R¹⁸ each independently represents a hydrogen atom or a substituent.
[2] The optical information recording medium as described in [1], wherein the recording layer comprises at least one platinum porphyrin represented by formula (I) and both the maximum peak wavelength in the solution absorption spectrum and the maximum peak wavelength in the film absorption spectrum of the platinum porphyrin represented by formula (I) are from 350 to 385 nm.
[3] The optical information recording medium as described in [1] or [2], wherein the recording layer comprises at least one platinum porphyrin represented by formula (I) and the refractive index n and extinction coefficient k of the recording layer at the oscillation wavelength of laser light of 440 nm or less satisfy n≥2.0 and 0.01≤k≤0.4 at the same time.
[4] The optical information recording medium as described in any one of [1] to [3], comprising a substrate having thereon a recording layer capable of recording information by the irradiation with laser light of 440 nm or less, wherein the recording layer comprises at least one platinum porphyrin represented by the following formula (II): Formula (II):

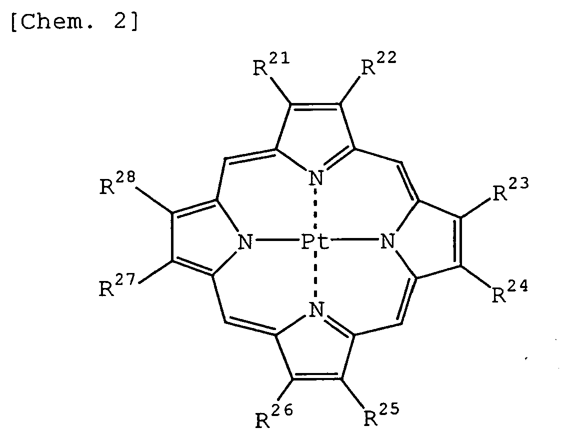

wherein R²¹ to R²⁸ each independently represents a substituted or unsubstituted alkyl group, provided that at least one substituent out of R²¹ to R²⁸ is a group further substituted by any one member of a hydroxyl group, a carboxyl group, an amino group, an alkylamino group, a mercapto group, an alkoxy group, an alkylthio group, an alkoxycarbonyl group, an alkylthiocarbonyl group, an alkylaminocarbonyl group and a heterocyclic group.
[5] The optical information recording medium as described in any one of [1] to [3], comprising a substrate having thereon a recording layer capable of recording information by the irradiation with laser light of 440 nm or less, wherein the recording layer comprises at least one platinum porphyrin represented by the following formula (III): Formula (III): 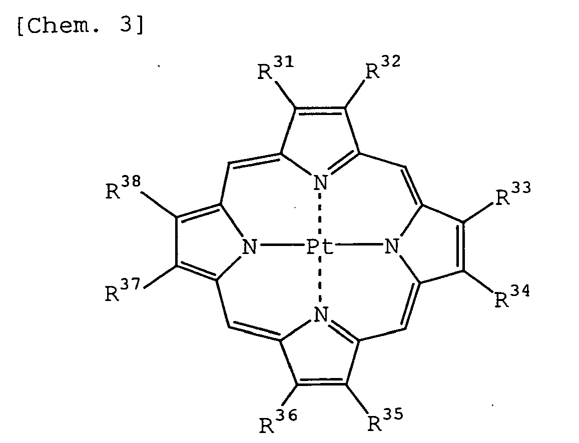 wherein R³¹ to R³⁸ each independently represents a substituted or unsubstituted alkoxy group or a substituted or unsubstituted alkylthio group, provided that at least one substituent out of R³¹ to R³⁸ is a group further substituted by any one member of a hydroxyl group, a carboxyl group, an amino group, an alkylamino group, a mercapto group, an alkoxy group, an alkylthio group, an alkoxycarbonyl group, an alkylthiocarbonyl group, an alkylaminocarbonyl group and a heterocyclic group.
[6] The optical information recording medium as described in any one of [1] to [5], wherein a light-reflecting layer comprising a metal is provided separately from the recording layer.
[7] The optical information recording medium as described in any one of [1] to [6], wherein a protective layer is provided separately from the recording layer.
[8] The optical information recording medium as described in any one of [1] to [7], wherein the substrate is a transparent disc-like substrate having on the surface thereof pregrooves at a track pitch of 0.2 to 0.5 mm and the recording layer is provided on the pregroove-formed surface.

### EFFECTS OF THE INVENTION

The optical information recording medium of the present invention uses a specific platinum porphyrin compound as the recording material of the recording layer and thereby produces an effect of allowing for high-density recording and reproduction of information by the irradiation with short-wavelength laser light of 440 nm or less, particularly, laser light in the vicinity of 405 nm having high versatility, and exhibiting good recording and reproducing characteristics such as high degree of modulation. That is, information can be recorded at a higher density than in the related-art CD-R or DVD-R and furthermore, a larger volume of information can be recorded.

### BRIEF DESCRIPTION OF THE DRAWINGS

[Fig. 1] A 2,2,3,3-tetrafluoropropanol solution absorption spectrum of Compound (3).
[Fig. 2] A film absorption spectrum of Compound (3).
[Fig. 3] A chloroform solution absorption spectrum of Compound (E).
[Fig. 4] A vapor-deposition film absorption spectrum of Compound (E).
[Fig. 5] A 2,2,3,3-tetrafluoropropanol solution absorption spectrum of Compound (B).
[Fig. 6] A film absorption spectrum of Compound (B).

### BEST MODE FOR CARRYING OUT THE INVENTION

The embodiments and compounds of the optical information recording medium and information recording method of the present invention are described in detail below.

The optical information recording medium of the present invention is an optical recording medium comprising a substrate having thereon a recording layer capable of recording information by the irradiation with laser light of 440 nm or less, wherein the recording layer comprises at least one platinum porphyrin (dye) represented by formula (I).

In formula (I) representing the porphyrin-based compound for use in the present invention, R¹¹ to R¹⁸ each independently represents hydrogen atom or a substituent. Examples of the substituent include a halogen atom, an alkyl group (including a cycloalkyl group and a bicycloalkyl group), an alkenyl group (including a cycloalkenyl group and a bicycloalkenyl group), an alkynyl group, an aryl group, a heterocyclic group, a cyano group, a hydroxyl group, a nitro group, a carboxyl group, an alkoxy group, an aryloxy group, a silyloxy group, a heterocyclic oxy group, an acyloxy group, a carbamoyloxy group, an alkoxycarbonyloxy group, an aryloxycarbonyloxy group, an amino group (including an alkylamino group and an arylamino group), an acylamino group, an aminocarbonylamino group, an alkoxycarbonylamino group, an aryloxycarbonylamino group, a sulfamoylamino group, an alkyl- or aryl-sulfonylamino group, a mercapto group, an alkylthio group, an arylthio group, a heterocyclic thio group, a sulfamoyl group, a sulfo group, an alkyl- or aryl-sulfinyl group, an alkyl- or aryl-sulfonyl group, an acyl group, an aryloxycarbonyl group, an alkoxycarbonyl group, a carbamoyl group, an aryl- or heterocyclic-azo group, an imido group, a phosphino group, a phosphinyl group, a phosphinyloxy group, a phosphinylamino group and a silyl group. The pair of R¹¹ and R¹², R¹³ and R¹⁴, R¹⁵ and R¹⁶, or R¹⁷ and R¹⁸ may combine with each other.

More specifically, R¹¹ to R¹⁸ each represents a halogen atom (e.g., chlorine, bromine, iodine), an alkyl group [a linear, branched or cyclic, substituted or unsubstituted alkyl group; the alkyl group includes an alkyl group (preferably an alkyl group having a carbon number of 1 to 30, e.g., methyl, ethyl, n-propyl, isopropyl, tert-butyl, n-octyl, eicosyl, 2-chloroethyl, 2-cyanoethyl, 2-ethylhexyl), a cycloalkyl group (preferably a substituted or unsubstituted cycloalkyl group having a carbon number of 3 to 30, e.g., cyclohexyl, cyclopentyl, 4-n-dodecylcyclohexyl), a bicycloalkyl group (preferably a substituted or unsubstituted bicycloalkyl group having a carbon number of 5 to 30, that is, a monovalent group obtained by removing one hydrogen atom from a bicycloalkane having a carbon number of 5 to 30, e.g., bicyclo[1,2,2]heptan-2-yl, bicyclo[2,2,2]octan-3-yl), and a tricyclic structure having many cyclic structures; the alkyl group in the substituents described below (for example, an alkyl group in an alkylthio group) also indicates an alkyl group having such a concept], an alkenyl group [a linear, branched or cyclic, substituted or unsubstituted alkenyl group; the alkenyl group includes an alkenyl group (preferably a substituted or unsubstituted alkenyl group having a carbon number of 2 to 30, e.g., vinyl, allyl, prenyl, geranyl, oleyl), a cycloalkenyl group (preferably a substituted or unsubstituted cycloalkenyl group having a carbon number of 3 to 30, that is, a monovalent group obtained by removing one hydrogen atom from a cycloalkene having a carbon number of 3 to 30, e.g., 2-cyclopenten-1-yl, 2-cyclohexen-1-yl), a bicycloalkenyl group (a substituted or unsubstituted bicycloalkenyl group, preferably a substituted or unsubstituted bicycloalkenyl group having a carbon number of 5 to 30, that is, a monovalent group obtained by removing one hydrogen atom from a bicycloalkene having one double bond, e.g., bicyclo[2,2,1]hept-2-en-1-yl, bicyclo[2,2,2]oct-2-en-4-yl)], an alkynyl group (preferably a substituted or unsubstituted alkynyl group having a carbon number of 2 to 30, e.g., ethynyl, propargyl, trimethylsilylethynyl), an aryl group (preferably a substituted or unsubstituted aryl group having a carbon number of 6 to 30, e.g., phenyl, p-tolyl, naphthyl, m-chlorophenyl, o-hexadecanoylaminophenyl), a heterocyclic group (preferably a monovalent group obtained by removing one hydrogen atom from a 5- or 6-membered substituted or unsubstituted, aromatic or non-aromatic heterocyclic compound, more preferably a 5- or 6-membered aromatic heterocyclic group having a carbon number of 3 to 30, e.g., 2-furyl, 2-thienyl, 2-pyrimidinyl, 2-benzothiazolyl), a cyano group, a hydroxyl group, a nitro group, a carboxyl group, an alkoxy group (preferably a substituted or unsubstituted alkoxy group having a carbon number of 1 to 30, e.g., methoxy, ethoxy, isopropoxy, tert-butoxy, n-octyloxy, 2-methoxyethoxy), an aryloxy group (preferably a substituted or unsubstituted aryloxy group having a carbon number of 6 to 30, e.g., phenoxy, 2-methylphenoxy, 4-tert-butylphenoxy, 3-nitrophenoxy, 2-tetradecanoylaminophenoxy), a silyloxy group (preferably a silyloxy group having a carbon number of 3 to 20, e.g., trimethylsilyloxy, tert-butyldimethylsilyloxy), a heterocyclic oxy group (preferably a substituted or unsubstituted heterocyclic oxy group having a carbon number of 2 to 30, e.g., 1-phenyltetrazol-5-oxy, 2-tetrahydropyranyloxy), an acyloxy group (preferably a formyloxy group, a substituted or unsubstituted alkylcarbonyloxy group having a carbon number of 2 to 30, or a substituted or unsubstituted arylcarbonyloxy group having a carbon number of 6 to 30, e.g., formyloxy, acetyloxy, pivaloyloxy, stearoyloxy, benzoyloxy, p-methoxyphenylcarbonyloxy), a carbamoyloxy group (preferably a substituted or unsubstituted carbamoyloxy group having a carbon number of 1 to 30, e.g., N,N-dimethylcarbamoyloxy, N,N-diethylcarbamoyloxy, morpholinocarbonyloxy, N,N-di-n-octylaminocarbonyloxy, N-n-octylcarbamoyloxy), an alkoxycarbonyloxy group (preferably a substituted or unsubstituted alkoxycarbonyloxy group having a carbon number of 2 to 30, e.g., methoxycarbonyloxy, ethoxycarbonyloxy, tert-butoxycarbonyloxy, n-octylcarbonyloxy), an aryloxycarbonyloxy group (preferably a substituted or unsubstituted aryloxycarbonyloxy group having a carbon number of 7 to 30, e.g., phenoxycarbonyloxy, p-methoxyphenoxycarbdnyloxy, p-n-hexadecyloxyphenoxycarbonyloxy), an amino group, an alkylamino group (preferably a substituted or unsubstituted alkylamino group having a carbon number of 1 to 30, e.g., methylamino, dimethylamino), an arylamino group (a substituted or unsubstituted anilino group having a carbon number of 6 to 30, e.g., anilino, N-methylanilino, diphenylamino), an acylamino group (preferably a formylamino group, a substituted or unsubstituted alkylcarboriylamino group having a carbon number of 1 to 30, or a substituted or unsubstituted arylcarbonylamino group having a carbon number of 6 to 30, e.g., formylamino, acetylamino, pivaloylamino, lauroylamino, benzoylamino, 3,4,5-tri-n-octyloxyphenylcarbonylamino), an aminocarbonylamino group (preferably a substituted or unsubstituted aminocarbonylamino group having a carbon number of 1 to 30, e.g., carbamoylamino, N,N-dimethylaminocarbonylamino, N,N-diethylaminocarbonylamino, morpholinocarbonylamino), an alkoxycarbonylamino group (preferably a substituted or unsubstituted alkoxycarbonylamino group having a carbon number of 2 to 30, e.g., methoxycarbonylamino, ethoxycarbonylamino, tert-butoxycarbonylamino, n-octadecyloxycarbonylamino, N-methylmethoxycarbonylamino), an aryloxycarbonylamino group (preferably a substituted or unsubstituted aryloxycarbonylamino group having a carbon number of 7 to 30, e.g., phenoxycarbonylamino, p-chlorophenoxycarbonylamino, m-n-octyloxyphenoxycarbonylamino), a sulfamoylamino group (preferably a substituted or unsubstituted sulfamoylamino group having a carbon number of 0 to 30, e.g., sulfamoylamino, N,N-dimethylaminosulfonylamino, N-n-octylaminosulfonylamino), an alkyl- or aryl-sulfonylamino group (preferably a substituted or unsubstituted alkylsulfonylamino group having a carbon number of 1 to 30, or a substituted or unsubstituted arylsulfonylamino group having a carbon number of 6 to 30, e.g., methylsulfonylamino, butylsulfonylamino, phenylsulfonylamino, 2,3,5-trichlorophenylsulfonylamino, p-methylphenylsulfonylamino), a mercapto group, an alkylthio group (preferably a substituted or unsubstituted alkylthio group having a carbon number of 1 to 30, e.g., methylthio, ethylthio, n-hexadecylthio), an arylthio group (preferably a substituted or unsubstituted arylthio group having a carbon number of 6 to 30, e.g., phenylthio, p-chlorophenylthio, m-methoxyphenylthio), a heterocyclic thio group (preferably a substituted or unsubstituted heterocyclic thio group having a carbon number of 2 to 30, e.g., 2-benzothiazolylthio, 1-phenyltetrazol-5-ylthio), a sulfamoyl group (preferably a substituted or unsubstituted sulfamoyl group having a carbon number of 0 to 30, e.g., N-ethylsulfamoyl, N-(3-dodecyloxypropyl)sulfamoyl, N,N-dimethylsulfamoyl, N-acetylsulfamoyl, N-benzoylsulfamoyl, N-(N'-phenylcarbamoyl)sulfamoyl), a sulfo group, an alkyl- or aryl-sulfinyl group (preferably a substituted or unsubstituted alkylsulfinyl group having a carbon number of 1 to 30, or a substituted or unsubstituted arylsulfinyl group having a carbon number of 6 to 30, e.g., methylsulfinyl, ethylsulfinyl, phenylsulfinyl, p-methylphenylsulfinyl), an alkyl- or aryl-sulfonyl group (preferably a substituted or unsubstituted alkylsulfonyl group having a carbon number of 1 to 30, or a substituted or unsubstituted arylsulfonyl group having a carbon number of 6 to 30, e.g., methylsulfonyl, ethylsulfonyl, tert-butylsulfonyl, s-butylsulfonyl, phenylsulfonyl, p-methylphenylsulfonyl), an acyl group (preferably a formyl group, a substituted or unsubstituted alkylcarbonyl group having a carbon number of 2 to 30, a substituted or unsubstituted arylcarbonyl group having a carbon number of 7 to 30, or a substituted or unsubstituted heterocyclic carbonyl group having a carbon number of 4 to 30 bonded to a carbonyl group via a carbon atom, e.g., acetyl, pivaloyl, 2-chloroacetyl, stearoyl, benzoyl, p-n-octyloxyphenylcarbonyl, 2-pyridylcarbonyl, 2-furylcarbonyl), an aryloxycarbonyl group (preferably a substituted or unsubstituted aryloxycarbonyl group having a carbon number of 7 to 30, e.g., phenoxycarbonyl, o-chlorophenoxycarbonyl, m-nitrophenoxycarbonyl, p-tert-butylphenoxycarbonyl), an alkoxycarbonyl group (preferably a substituted or unsubstituted alkoxycarbonyl group having a carbon number of 2 to 30, e.g., methoxycarbonyl, ethoxycarbonyl, tert-butoxycarbonyl, n-octadecyloxycarbonyl), a carbamoyl group (preferably a substituted or unsubstituted carbamoyl group having a carbon number of 1 to 30, e.g., carbamoyl, N-methylcarbamoyl, N,N-dimethylcarbamoyl, N,N-di-n-octylcarbamoyl, N-(methylsulfonyl)carbamoyl), an aryl- or heterocyclic-azo group (preferably a substituted or unsubstituted arylazo group having a carbon number of 6 to 30, or a substituted or unsubstituted heterocyclic azo group having a carbon number of 3 to 30, e.g., phenylazo, p-chlorophenylazo, 5-ethylthio-1,3,4-thiadiazol-2-ylazo), an imido group (preferably N-succinimido or N-phthalimido), a phosphino group (preferably a substituted or unsubstituted phosphino group having a carbon number of 2 to 30, e.g., dimethylphosphino, diphenylphosphino, methylphenoxyphosphino), a phosphinyl group (preferably a substituted or unsubstituted phosphinyl group having a carbon number of 2 to 30, e.g., phosphinyl, dioctyloxyphosphinyl, diethoxyphosphinyl), a phosphinyloxy group (preferably a substituted or unsubstituted phosphinyloxy group having a carbon number of 2 to 30, e.g., diphenoxyphosphinyloxy, dioctyloxyphosphinyloxy), a phosphinylamino group (preferably a substituted or unsubstituted phosphinylamino group having a carbon number of 2 to 30, e.g., dimethoxyphosphinylamino, dimethylaminophosphinylamino), or a silyl group (preferably a substituted or unsubstituted silyl group having a carbon number of 3 to 30, e.g., trimethylsilyl, tert-butyldimethylsilyl, phenyldimethylsilyl).

Of these functional groups, those having a hydrogen atom may be further substituted with a group described above by removing the hydrogen atom. Examples of such a functional group include an alkylcarbonylaminosulfonyl group, an arylcarbonylaminosulfonyl group, an alkylsulfonylaminocarbonyl group and an arylsulfonylaminocarbonyl group. Examples thereof include methylsulfonylaminocarbonyl, p-methylphenylsulfonylaminocarbonyl, acetylaminosulfonyl and benzoylaminosulfonyl. R¹¹ to R¹⁸ each may be further substituted by a substituent.

R¹¹ to R¹⁸ each is preferably a halogen atom, a substituted or unsubstituted alkyl group having a carbon number of 1 to 30, a substituted or unsubstituted aryl group having a carbon number of 6 to 30, a 5- or 6-membered substituted or unsubstituted heterocyclic group, a cyano group, a substituted or unsubstituted alkylamino group having a carbon number of 1 to 30, a substituted or unsubstituted arylamino group having a carbon number of 6 to 30, a substituted or unsubstituted alkoxy group having a carbon number of 1 to 30, a substituted or unsubstituted alkylcarbonyl group having a carbon number of 2 to 30, a substituted or unsubstituted arylcarbonyl group having a carbon number of 7 to 30, a substituted or unsubstituted alkoxycarbonyl group having a carbon number of 2 to 30, a substituted or unsubstituted aryloxycarbonyl group having a carbon number of 7 to 30, a substituted or unsubstituted alkylsulfonyl group having a carbon number of 1 to 30, or a substituted or unsubstituted arylsulfonyl group having a carbon number of 6 to 30, more preferably an alkyl group having a carbon number of 1 to 30, a substituted or unsubstituted alkylamino group having a carbon number of 1 to 30, a substituted or unsubstituted arylamino group having a carbon number of 6 to 30, a substituted or unsubstituted alkoxy group having a carbon number of 1 to 30, a substituted or unsubstituted alkylcarbonyl group having a carbon number of 2 to 30, a substituted or unsubstituted alkoxycarbonyl group having a carbon number of 2 to 30, or a substituted or unsubstituted alkylthio group having a carbon number of 1 to 30, still more preferably a substituted or unsubstituted alkyl group having a carbon number of 1 to 30, a substituted or unsubstituted alkylamino group having a carbon number of 1 to 30, a substituted or unsubstituted alkoxy group having a carbon number of 1 to 30, or a substituted or unsubstituted alkyl thio group having a carbon number of 1 to 30.

The preferred embodiment of the compound represented by formula (I) for use in the present invention is formula (II) or formula (III), more preferably formula (III).

Formula (II) is described below. R²¹ to R²⁸ each independently represents a substituted or unsubstituted alkyl group having a carbon number of 1 to 30, provided that at least one substituent out of R²¹ to R²⁸ is an alkyl group further substituted by any one of a hydroxyl group, a carboxyl group, an amino group, an alkylamino group, a mercapto group, an alkoxy group, an alkylthio group, an alkoxycarbonyl group, an alkylthiocarbonyl group, an alkylaminocarbonyl group and a heterocyclic group.

At least one substituent out of R²¹ to R²⁸ is an alkyl group substituted by any one of a hydroxyl group, a carboxyl group, an unsubstituted amino group, a substituted or unsubstituted alkylamino group, a mercapto group, a substituted or unsubstituted alkoxy group, a substituted or unsubstituted alkylthio group, a substituted or unsubstituted alkoxycarbonyl group, a substituted or unsubstituted alkylthiocarbonyl group, a substituted or unsubstituted alkylaminocarbonyl group and a substituted or unsubstituted heterocyclic group, preferably an alkyl group substituted by any one of a substituted or unsubstituted alkoxy group, a substituted or unsubstituted alkylthio group, a substituted or unsubstituted alkylamino group and a substituted or unsubstituted heterocyclic group, more preferably an alkyl group substituted by any one of a substituted or unsubstituted alkoxy group, a substituted or unsubstituted alkylthio group and a substituted or unsubstituted alkylamino group, still more preferably an alkyl group substituted by any one of a substituted or unsubstituted alkoxy group and a substituted or unsubstituted alkylthio group, yet still more preferably an alkyl group substituted by a substituted or unsubstituted alkoxy group.

Formula (III) is described below. R³¹ to R³⁸ each independently represents a substituted or unsubstituted alkoxy group having a carbon number of 1 to 30 or a substituted or unsubstituted alkylthio group having a carbon number of 1 to 30, provided that at least one substituent out of R³¹ to R³⁸ is a group further substituted by any one of a hydroxyl group, a carboxyl group, an amino group, an alkylamino group, a mercapto group, an alkoxy group, an alkylthio group, an alkoxycarbonyl group, an alkylthiocarbonyl group, an alkylaminocarbonyl group and a heterocyclic group.

At least one substituent out of R³¹ to R³⁸ is a group (an alkoxy group or an alkylthio group) substituted by any one of a hydroxyl group, a carboxyl group, an unsubstituted amino group, a substituted or unsubstituted alkylamino group, a mercapto group, a substituted or unsubstituted alkoxy group, a substituted or unsubstituted alkylthio group, a substituted or unsubstituted alkoxycarbonyl group, a substituted or unsubstituted alkylthiocarbonyl group, a substituted or unsubstituted alkylaminocarbonyl group and a substituted or unsubstituted heterocyclic group, preferably a group substituted by any one of a substituted or unsubstituted alkoxy group, a substituted or unsubstituted alkylthio group, a substituted or unsubstituted alkylamino group and a substituted or unsubstituted heterocyclic group, more preferably a group substituted by any one of a substituted or unsubstituted alkoxy group, a substituted or unsubstituted alkylthio group and a substituted or unsubstituted alkylamino group, still more preferably a group substituted by any one of a substituted or unsubstituted alkoxy group and a substituted or unsubstituted alkylthio group, yet still more preferably a group substituted by a substituted or unsubstituted alkoxy group.

In the platinum porphyrin represented by formula (I), the maximum peak wavelength of the solution absorption spectrum and the maximum peak wavelength of the film absorption spectrum both are from 350 to 385 nm. The wavelength range for the maximum peak wavelength of the solution absorption spectrum and the maximum peak wavelength of the film absorption spectrum both is preferably from 360 to 380 nm, more preferably from 365 to 380 nm. The reason therefor is that the refractive index n takes a larger value, a high degree of modulation is liable to result, and good recording characteristics are obtained. The solvent used for the measurement of the absorption spectrum may be any solvent but is preferably 2,2,3,3-tetrafluoropropanol or chloroform. In the present invention, a value obtained by using 2,2,3,3-tetrafluoropropanol or chloroform is employed. The film used for the measurement of the film absorption spectrum may be any film as long as it contains platinum porphyrin, but this is a film formed by coating a solution (preferably a solvent, for example, 2,2,3,3-tetrafluoropropanol) of platinum porphyrin by spin coating or the like and may be a platinum porphyrin-containing recording layer of an optical information recording medium. The film may be in the crystal state or amorphous state but is preferably in the amorphous state. The thickness of the film is not particularly limited but in the present invention, a film thickness of 10 to 300 nm is employed.

In the recording layer containing at least one platinum porphyrin represented by formula (I), the refractive index n and the extinction coefficient k at the oscillation wavelength of laser light of 440 nm must satisfy n≥2.0 and 0.01≤k≤0.4 at the same time.

The refractive index n and the extinction coefficient k at the oscillation wavelength of laser light of 440 nm preferably satisfy n≥2.1 and 0.1≤k≤0.4 at the same time, more preferably n≥2.1 and 0.25≤k≤0.4 at the same time, still more preferably n≥2.1 and 0.3≤k≤0.4 at the same time. Within these preferred ranges, the degree of modulation becomes large and good recording characteristics are obtained.

Specific preferred examples of the porphyrin-based compound for use in the present invention are set forth below, but the present invention is not limited thereto.

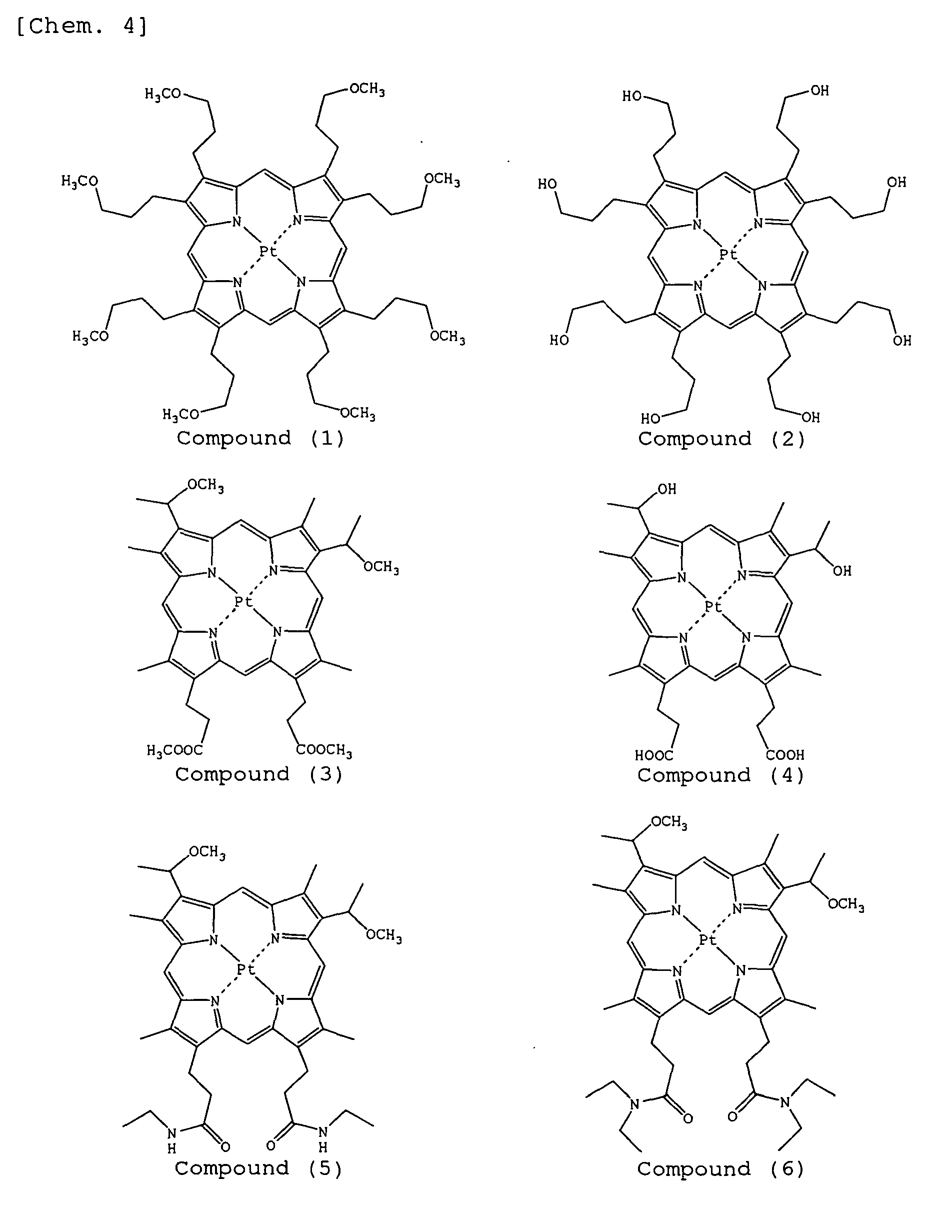

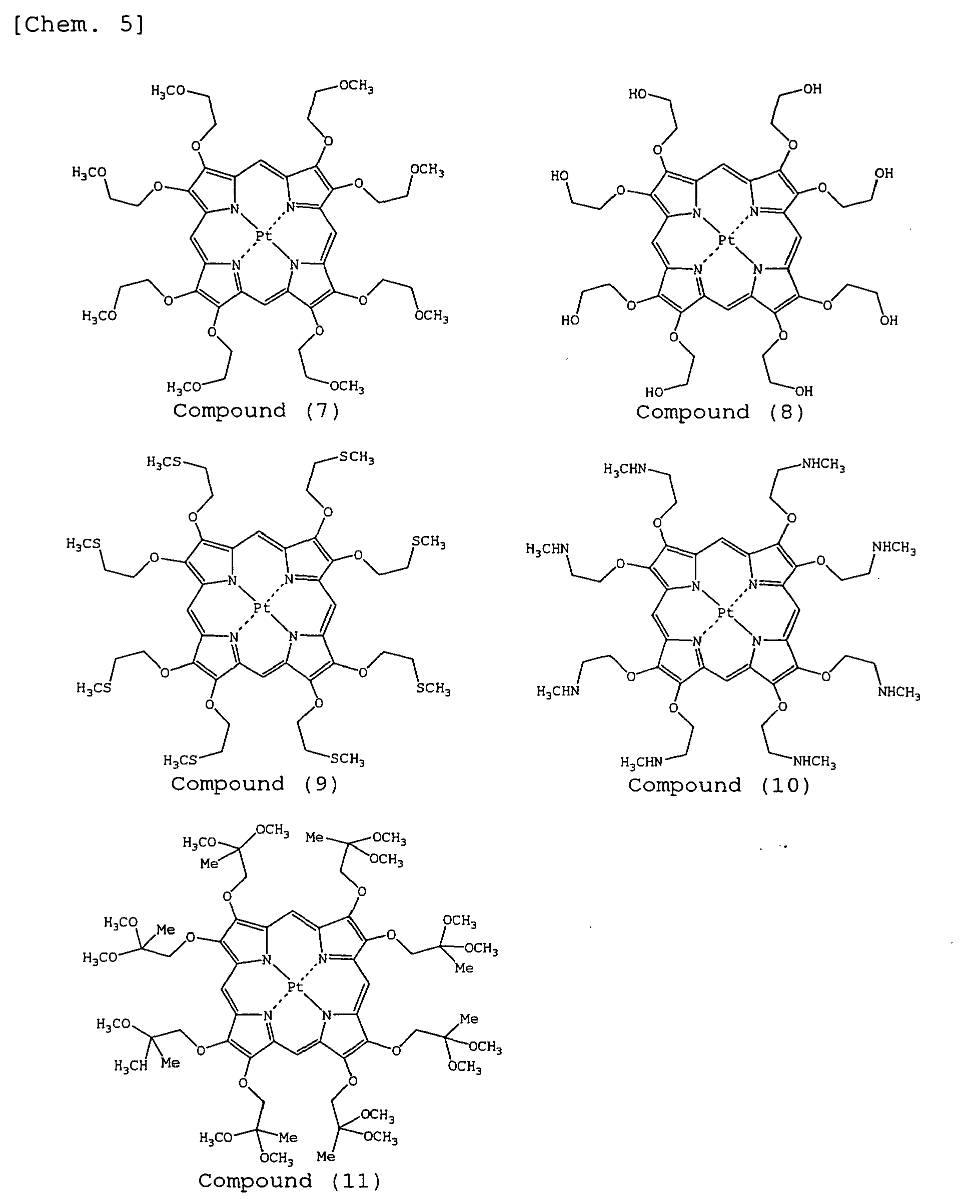

Comparative compounds with the present invention are set forth below.

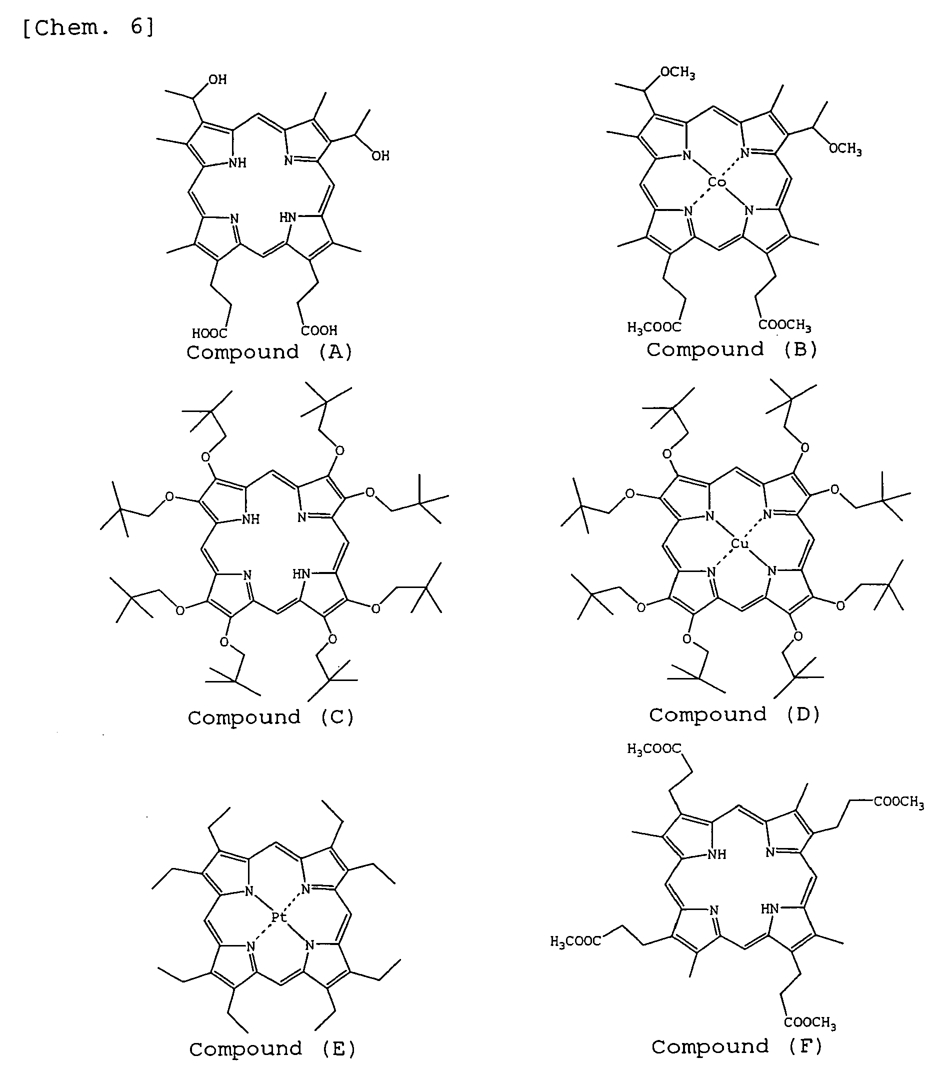

### <Optical Information Recording Medium>

The optical information recording medium of the present invention is preferably an embodiment [1]: an optical information recording medium having a dye-containing write-once recording layer and a cover layer having a thickness of 0.01 to 0.5 mm in this order on a substrate having a thickness of 0.7 to 2 mm, or an embodiment [2]: an optical information recording medium having a dye-containing write-once recording layer and a protective substrate having a thickness of 0.1 to 1.0 mm in this order on a substrate having a thickness of 0.1 to 1.0 mm. In the embodiment [1], it is preferred that the track pitch of pregrooves formed on the substrate is from 50 to 500 nm, the groove width is from 25 to 250 nm and the groove depth is from 5 to 150 nm. In the embodiment [2], it is preferred that the track pitch of pregrooves formed on the substrate is from 200 to 600 nm, the groove width is from 50 to 300 nm, the groove depth is from 30 to 200 nm, and the wobble amplitude is from 10 to 50 nm.
The optical information recording medium of the embodiment [1] is an embodiment having at least a substrate, a write-once recording layer and a cover layer. These essential members are sequentially described below.

### [Substrate of Embodiment [1]]

On the substrate of the preferred embodiment [1], a pregroove (guide groove) having a shape with a track pitch, a groove width (half-value width), a groove depth and a wobble amplitude all in the following ranges must be formed. This pregroove is provided to achieve a higher recording density as compared with CD-R and DVD-R and is suitable, for example, when using the optical information recording medium as a medium responding to a blue-violet laser.

The track pitch of the pregrooves must be from 200 to 500 nm, and the lower limit is preferably 420 nm or less, more preferably 370 nm or less, still more preferably 330 nm or less. Also, the lower limit is preferably 260 nm or more.
If the track pitch is less than 200 nm, the pregroove is difficult to exactly form and a problem of crosstalk may arise, whereas if it exceeds 500 nm, the recording density may be disadvantageously decreased.

The groove width (half-value width) of the pregroove must be from 25 to 250 nm. The upper limit is preferably 200 nm or less, more preferably 170 nm or less, still more preferably 150 nm or less. The lower limit is preferably 50 nm or more, more preferably 80 nm or more, still more preferably 100 nm or more.
If the groove width of the pregroove is less than 25 nm, the groove may not be satisfactorily transferred at the molding or the error rate of recording may be increased, whereas if it exceeds 250 nm, the pit formed at the recording is enlarged and this may give rise to crosstalk or failure in obtaining a sufficiently large degree of modulation.

The groove depth of the pregroove must be from 5 to 150 nm. The upper limit is preferably 100 nm or less, more preferably 70 nm or less, still more preferably 50 nm or less. The lower limit is preferably 10 nm or more, more preferably 20 nm or more, still more preferably 28 nm or more.
If the groove depth of the pregroove is less than 5 nm, a sufficiently large degree of recording modulation may not be obtained, whereas if it exceeds 150 nm, the reflectance sometimes greatly decreases.

As for the groove tilt angle of the pregroove, the upper limit is preferably 80° or less, more preferably 75° or less, still more preferably 70° or less, yet still more preferably 65° or less, and the lower limit is preferably 20° or more, more preferably 30° or more, still more preferably 40° or more.
If the groove tilt angle of the pregroove is less than 20°, a sufficiently large tracking error signal amplitude may not be obtained, whereas if it exceeds 80°, the molding becomes difficult.

The substrate for use in the present invention may be arbitrarily selected from various materials used as the substrate material of conventional optical information recording mediums.
Specific examples thereof include glass; an acrylic resin such as polycarbonate and polymethyl methacrylate; a vinyl chloride-based resin such as polyvinyl chloride and vinyl chloride copolymer; an epoxy resin, an amorphous polyolefin; a polyester; and a metal such as aluminum. Some of these materials may be used in combination, if desired.
Among the materials described above, a thermoplastic resin such as amorphous polyolefin and polycarbonate is preferred in view of moisture resistance, dimensional stability, cost and the like, and a polycarbonate is more preferred.
When using such a resin, the substrate may be produced by using an injection mold.
The thickness of the substrate needs to be from 0.7 to 2 mm and is preferably from 0.9 to 1.6 mm, more preferably from 1.0 to 1.3 mm.

On the substrate surface on the side where a light-reflecting layer describe later is provided, an undercoat layer is preferably formed for the purpose of improving the planarity and enhancing the adhesive strength.
Examples of the material for the undercoat layer include a polymer material such as polymethyl methacrylate, acrylic acid-methacrylic acid copolymer, styrene-maleic anhydride copolymer, polyvinyl alcohol, N-methylolacrylamide, styrene-vinyl toluene copolymer, chlorosulfonated polyethylene, nitrocellulose, polyvinyl chloride, chlorinated polyolefin, polyester, polyimide, vinyl acetate-vinyl chloride copolymer, ethylene-vinyl acetate copolymer, polyethylene, polypropylene and polycarbonate; and a surface modifier such as silane coupling agent.
The undercoat layer may be formed by dissolving or dispersing the above-described material in an appropriate solvent to prepare a coating solution, and then applying the coating solution to the substrate surface according to a coating method such as spin coating, dip coating and extrusion coating. The thickness of the undercoat layer is generally from 0.005 to 20 µm, preferably from 0.01 to 10 µm.

### [Write-Once Recording Layer of Embodiment [1]]

The write-once recording layer of the preferred embodiment [1] is formed by dissolving a dye together with a binder and the like in an appropriate solvent to prepare a coating solution, applying this coating solution to a substrate or a light-reflecting layer described later to form a coating film, and drying the coating film. The write-once recording layer may be a single layer or may have a multilayer structure. In the case of a multilayer structure, the step of applying the coating solution is preformed a plurality of times.
The dye concentration in the coating solution is generally from 0.01 to 15 mass%, preferably from 0.1 to 10 mass%, more preferably from 0.5 to 5 mass%, and most preferably from 0.5 to 3 mass%.

Examples of the solvent for the coating solution include an ester such as butyl acetate, ethyl lactate and cellosolve acetate; a ketone such as methyl ethyl ketone, cyclohexanone and methyl isobutyl ketone; a chlorinated hydrocarbon such as dichloromethane, 1,2-dichloroethane and chloroform; an amide such as dimethylformamide; a hydrocarbon such as methylcyclohexane; an ether such as tetrahydrofuran, ethyl ether, diisopropyl ether, dibutyl ether and dioxane; an alcohol such as ethanol, n-propanol, isopropanol, n-butanol and diacetone alcohol; a fluorine-based solvent such as 2,2,3,3-tetrafluoropropanol, octafluoropentanol and hexafluorobutanol; and glycol ethers such as ethylene glycol monomethyl ether, ethylene glycol monoethyl ether and propylene glycol monomethyl ether.
Considering the solubility of the dye used, one of these solvents may be used alone or two or more species thereof may be used in combination. In the coating solution, various additives such as antioxidant, a UV absorbent, plasticizer and lubricant may be further added according to the purpose.

Examples of the coating method include a spray method, a spin coating method, a dipping method, a roll coating method, a blade coating method, a doctor roll method and a screen printing method.
At the coating, the temperature of the coating solution is preferably from 23 to 50°C, more preferably from 24 to 40°C, still more preferably from 24 to 37°C.

The thickness of the thus-formed write-once recording layer is, on the groove (convex of the substrate above), preferably 300 nm or less, more preferably 250 nm or less, still more preferably 200 nm or less, yet still more preferably 180 nm or less. The lower limit is preferably 30 nm or more, more preferably 50 nm or more, still more preferably 70 nm or more, yet still more preferably 90 nm or more.
Also, the thickness of the write-once recording layer is, on the land (concave of the substrate above), preferably 400 nm or less, more preferably 300 nm or less, still more preferably 250 nm or less. The lower limit is preferably 70 nm or more, more preferably 90 nm or more, still more preferably 110 nm or more.
The ratio of thickness of the write-once recording layer on the groove/thickness of the write-once recording layer on the land is preferably 0.4 or more, more preferably 0.5 or more, still more preferably 0.6 or more, yet still more preferably 0.7 or more. The upper limit is preferably less than 1, more preferably 0.9 or less, still more preferably 0.85 or less, yet still more preferably 0.8 or less.

In the case where the coating solution contains a binder, examples of the binder include a natural organic polymer substance such as gelatin, cellulose derivative, dextran, rosin and rubber; and a synthetic organic polymer including a hydrocarbon-based resin such as polyethylene, polypropylene, polystyrene and polyisobutylene, a vinyl-based resin such as polyvinyl chloride, polyvinylidene chloride and polyvinyl chloride-polyvinyl acetate copolymer, an acrylic resin such as polymethyl acrylate and polymethyl methacrylate, a polyvinyl alcohol, a chlorinated polyethylene, an epoxy resin, a butyral resin, a rubber derivative, and an initial condensate of thermosetting resin such as phenol-formaldehyde resin. In the case of using a binder in combination as the material of the write-once recording layer, the amount of the binder used is generally from 0.01 to 50 times (by mass), preferably from 0.1 to 5 times (by mass), based on the dye.

Furthermore, in the write-once recording layer, various discoloration inhibitors may be incorporated so as to more enhance the light fastness of the write-once recording layer. The discoloration inhibitor generally used is a singlet oxygen quencher. Also in the present invention, when this singlet oxygen quencher is mixed, more enhancement of the light fastness can be expected. As for the single oxygen quencher, those described in already published patent applications can be used.
The amount used of the discoloration inhibitor such as singlet oxygen quencher is generally from 0.1 to 50 mass%, preferably from 0.5 to 45 mass%, more preferably from 3 to 40 mass%, still more preferably from 5 to 25 mass%, based on the amount of the dye.

### [Cover Layer of Embodiment [1]]

The cover layer of the preferred embodiment [1] is laminated on the above-described write-once recording layer or a barrier layer described later through an adhesive or a pressure-sensitive adhesive.
The cover layer for use in the present invention is not particularly limited as long as it is a film formed of a transparent material, but, for example, an acrylic resin such as polycarbonate and polymethyl methacrylate; a vinyl chloride-based resin such as polyvinyl chloride and vinyl chloride copolymer; an epoxy resin; an amorphous polyolefin; a polyester; a cellulose triacetate are preferably used. Among these, a polycarbonate and a cellulose triacetate are more preferred.
Incidentally, the term "transparent" means that the transmittance of light used for recording and reproduction is 80% or more.

Also, the cover layer may contain various additives in the range of not impairing the effects of the present invention. For example, a dye for cutting light of 500 nm or more may be incorporated.
As for the surface physical property of the cover layer, the surface roughness is preferably 5 nm or less in terms of both a two-dimensional roughness parameter and a three-dimensional roughness parameter.
Furthermore, in view of the condensing degree of light used for recording and reproduction, the cover film preferably has a birefringence of 10 nm or less.

The thickness of the cover layer is appropriately specified according to the wavelength of laser light irradiated for recording and reproduction or the NA but in the present invention, the thickness is from 0.01 to 0.5 mm, preferably from 0.05 to 0.12 mm.
The total thickness of the cover layer and a layer comprising an adhesive or a pressure-sensitive adhesive is preferably from 0.09 to 0.11 mm, more preferably from 0.095 to 0.105 mm.
Incidentally, on the light incident surface of the cover layer, a protective layer (hardcoat layer) may be provided for preventing the light incident surface from damaging during the production of the optical information recording medium.

The adhesive used for laminating the cover layer is preferably, for example, a UV curable resin, an EB curable resin or a thermosetting resin, more preferably a UV curable resin. In the case of using a UV curable resin as the adhesive, the UV curable resin may be used as it is or may be dissolved in an appropriate solvent such as methyl ethyl ketone and ethyl acetate to prepare a coating solution and supplied to the barrier layer surface from a dispenser. In order to prevent warpage of the produced optical information recording medium, the UV curable resin constituting the adhesive layer preferably has a curing shrinkage percentage. Examples of this UV curable resin include a UV curable resin such as "SD-640" produced by Dainippon Ink & Chemicals, Inc.

The adhesive in a predetermined amount is coated, for example, on a lamination surface comprising a barrier layer and after placing the cover layer thereon, the adhesive is preferably spread uniformly between the lamination surface and the cover layer by spin-coating and then cured.
The thickness of the adhesive layer comprising such an adhesive is preferably from 0.1 to 100 µm, more preferably from 0.5 to 50 µm, still more preferably from 10 to 30 µm.

The pressure-sensitive adhesive used for laminating the cover layer may be an acryl-based, rubber-based or silicon-based pressure-sensitive adhesive, but in view of transparency and durability, an acryl-based pressure-sensitive adhesive is preferred. The acryl-based pressure-sensitive adhesive is preferably a pressure-sensitive adhesive produced by using a 2-ethylhexyl acrylate, an n-butyl acrylate or the like as the main component and copolymerizing it with a short-chain alkyl acrylate or alkyl methacrylate such as methyl acrylate, ethyl acrylate or methyl methacrylate for enhancing the cohesive force, and an acrylic acid, a methacrylic acid, an acrylamide derivative, a maleic acid, a hydroxyethyl acrylate, a glycidyl acrylate or the like, which can work out to a crosslinking site with a crosslinking agent. The glass transition temperature (Tg) or the crosslinking density may be varied by appropriately controlling the mixing ratio and kind of the main component, the short-chain component and the component for adding a crosslinking site.

The crosslinking agent used in combination with the pressure-sensitive adhesive includes, for example, an isocyanate-based crosslinking agent. Examples of the isocyanate-based crosslinking agent which can be used include isocyanates such as tolylene diisocyanate, 4,4'-diphenylmethane diisocyanate, hexamethylene diisocyanate, xylylene diisocyanate, naphthylene-1,5-diisocyanate, o-toluidine isocyanate, isophorone diisocyanate and triphenylmethane triisocyanate; a product of these isocyanates with a polyalcohol; and polyisocyanates produced by the condensation of isocyanates. As for these isocyanates, examples of the commercially available product include Colonate L, Colonate HL, Colonate 2030, Colonate 2031, Millionate MR and Millionate HTL produced by Nippon Polyurethane Industry Co., Ltd.; Takenate D-102, Takenate D-110N, Takenate D-200 and Takenate D-202 produced by Takeda Chemical Industries, Ltd.; and Desmodule L, Desmodule IL, Desmodule N and Desmodule HL produced by Sumitomo Bayer Urethane Co., Ltd.

The pressure-sensitive adhesive may be cured after a predetermined amount of the pressure-sensitive adhesive is uniformly coated on the lamination surface comprising a barrier layer and the cover layer is placed thereon, or may be cured after a predetermined amount of the pressure-sensitive adhesive is uniformly coated in advance on one surface of the cover layer and the coating film is laminated with the lamination surface.
Furthermore, a commercially available pressure-sensitive adhesive film having previously provided thereon a pressure-sensitive adhesive layer may also be used for the cover layer. The thickness of the pressure-sensitive adhesive layer comprising a pressure-sensitive adhesive is preferably from 0.1 to 100 µm, more preferably from 0.5 to 50 µm, still more preferably from 10 to 30 µm.

### [Other Layers in Embodiment [1]]

The optical information recording medium of the preferred embodiment [1] may have other arbitrary layers in addition to the above-described essential layers, within the range of not impairing the effects of the present invention. Examples of other arbitrary layers include a label layer having a desired image, formed on the back surface of the substrate (back surface with respect to the surface on which the write-once recording layer is formed), a light-reflecting layer (described later) provided between the substrate and the write-once recording layer, a barrier layer (described later) provided between the write-once recording layer and the cover layer, and an interface layer provided between the light-reflecting layer and the write-once recording layer. Here, the label layer is formed by using an ultraviolet curable resin, a thermosetting resin, a heat-drying resin, or the like.
These essential and arbitrary layers each may be a single layer or may have a multilayer structure.

### [Light-Reflecting Layer in Embodiment [1]]

In the optical information recording medium of the preferred embodiment [1], a light-reflecting layer is preferably formed between the substrate and the write-once recording layer so as to impart a function of enhancing the reflectance for laser light or improving the recording and reproducing characteristics.
The light-reflecting layer may be formed on the substrate by vacuum-depositing, sputtering or ion-plating a light-reflecting substance having a high reflectance for laser light.
The thickness of the light-reflecting layer is generally from 10 to 300 nm, preferably from 50 to 200 nm.
The above-described reflectance is preferably 70% or more.

Examples of the light-reflecting substance having a high reflectance include a metal such as Mg, Se, Y, Ti, Zr, Hf, V, Nb, Ta, Cr, Mo, W, Mn, Re, Fe, Co, Ni, Ru, Rh, Pd, Ir, Pt, Cu, Ag, Au, Zn, Cd, Al, Ga, In, Si, Ge, Te, Pb, Po, Sn and Bi, a semimetal and a stainless steel. One of these light-reflecting substances may be used alone, or two or more species thereof may be used in combination or as an alloy. Among these, preferred are Cr, Ni, Pt, Cu, Ag, Au, Al and a stainless steel, more preferred are Au, Ag and Al and an alloy thereof, and most preferred are Au, Ag and an alloy thereof.

### [Formation Step of Barrier Layer (Intermediate Layer) in Embodiment [1]]

In the optical information recording medium of the preferred embodiment [1], a barrier layer is preferably formed between the write-once recording layer and the cover layer.
The barrier layer is provided, for example, to enhance the storability of the write-once recording layer, enhance the adhesion between the write-once recording layer and the cover layer, or adjust the reflectance or thermal conductivity.
The material used for the barrier layer is a material capable of transmitting light used for recording and reproduction and is not particularly limited as long as this function can be expressed, but in general, the material is a material having low permeability to gas or moisture and is preferably a dielectric material.
Specifically, a material comprising a nitride, an oxide, a carbide or a sulfide of Zn, Si, Ti, Te, Sn, Mo, Ge or the like is preferred. In particular, ZnS, MoO₂, GeO₂, TeO, SiO₂, TiO₂, ZuO, ZnS-SiO₂, SnO₂ and ZnO-Ga₂O₃ are preferred, and ZnS-SiO₂, SnO₂ and ZnO-Ga₂O₃ are more preferred.

The barrier layer can be formed by a vacuum film-forming method such as vacuum-deposition, DC sputtering, RF sputtering and ion-plating. Among these, sputtering is preferred, and RF sputtering is more preferred.
In the present invention, the thickness of the barrier layer is preferably from 1 to 200 nm, more preferably from 2 to 100 nm, still more preferably from 3 to 50 nm.

The optical information recording medium of the preferred embodiment [2] is described below.
The optical information recording medium of the embodiment [2] is an optical information recording medium having a lamination-type layer construction, and representative layer constructions are as follows.
(1) A first layer construction is a construction where a write-once recording layer, a light-reflecting layer and an adhesive layer are sequentially formed on a substrate, and a protective substrate is provided on the adhesive layer.
(2) A second layer construction is a construction where a write-once recording layer, a light-reflecting layer, a protective layer and an adhesive layer are sequentially formed on a substrate, and a protective substrate is provided on the adhesive layer.
(3) A third layer construction is a construction where a write-once recording layer, a light-reflecting layer, a protective layer, an adhesive layer and a protective layer are sequentially formed on a substrate, and a protective substrate is provided on the protective layer.
(4) A fourth layer construction is a construction where a write-once recording layer, a light-reflecting layer, a protective layer, an adhesive layer, a protective layer and a light-reflecting layer are sequentially formed on a substrate, and a protective substrate is provided on the light-reflecting layer.
(5) A fifth layer construction is a construction where a write-once recording layer, a light-reflecting layer, an adhesive layer and a light-reflecting layer are sequentially formed on a substrate, and a protective substrate is provided on the light-reflective layer.
   These layer constructions of (1) to (5) above are merely an example, and the layer construction may be changed in the order, may be partially replaced or may be partially omitted. Also, the write-once recording layer may be formed also on the protective substrate side and in this case, an optical information recording medium capable of recording and reproduction from both sides is obtained. Furthermore, each layer may comprise one layer or a plurality of layers.
   The optical information recording medium of the present invention is described below by referring, as an example, to an optical information recording medium in a construction having a write-once recording layer, a light-reflecting layer, an adhesive layer and a protective substrate in this order on a substrate.

### [Substrate of Embodiment [2]]

On the substrate of the preferred embodiment [2], a pregroove (guide groove) having a shape with a track pitch, a groove width (half-value width), a groove depth and a wobble amplitude all in the following ranges must be formed. This pregroove is provided to achieve a higher recording density as compared with CD-R and DVD-R and is suitable, for example, when using the optical information recording medium of the present invention as a medium responding to a blue-violet laser.

The track pitch of the pregrooves must be from 200 to 500 nm, and the upper limit is preferably 450 nm or less, more preferably 430 nm or less. Also, the lower limit is preferably 300 nm or more, more preferably 330 nm or more, still more preferably 370 nm or more.
If the track pitch is less than 200 nm, the pregroove is difficult to exactly form and a problem of crosstalk may arise, whereas if it exceeds 500 nm, the recording density may be disadvantageously decreased.

The groove width (half-value width) of the pregroove must be from 50 to 300 nm. The upper limit is preferably 250 nm or less, more preferably 200 nm or less, still more preferably 180 nm or less. The lower limit is preferably 100 nm or more, more preferably 120 nm or more, still more preferably 140 nm or more.
If the groove width of the pregroove is less than 50 nm, the groove may not be satisfactorily transferred at the molding or the error rate of recording may be increased, whereas if it exceeds 300 nm, the pit formed at the recording is enlarged and this may give rise to crosstalk or failure in obtaining a sufficiently large degree of modulation.

The groove depth of the pregroove must be from 30 to 200 nm. The upper limit is preferably 170 nm or less, more preferably 140 nm or less, still more preferably 120 nm or less. The lower limit is preferably 40 nm or more, more preferably 50 nm or more, still more preferably 60 nm or more.
If the groove depth of the pregroove is less than 30 nm, a sufficiently large degree of recording modulation may not be obtained, whereas if it exceeds 200 nm, the reflectance sometimes greatly decreases.

The substrate for use in the preferred embodiment [2] may be arbitrarily selected from various materials used as the substrate material of conventional optical information recording mediums. Specific examples and preferred examples thereof are the same as those for the substrate in the embodiment [1].
The thickness of the substrate needs to be from 0.1 to 1.0 mm and is preferably from 0.2 to 0.8 mm, more preferably from 0.3 to 0.7 mm.

On the substrate surface on the side where a write-once recording layer describe later is provided, an undercoat layer is preferably formed for the purpose of improving the planarity and enhancing the adhesive strength. Specific examples and preferred examples of the material for the undercoat layer, the coating method and the layer thickness are the same as those for the undercoat layer in the embodiment [1].

### [Write-Once Recording Layer of Embodiment [2]]

The detailed description of the write-once recording layer in the preferred embodiment [2] is the same as that for the write-once recording layer in the embodiment [1].

### [Light-Reflecting Layer of Embodiment [2]]

In the preferred embodiment [2], a light-reflecting layer is sometimes formed on the write-once recording layer so as to impart a function of enhancing the reflectance for laser light or improving the recording and reproducing characteristics. The details of the light-reflecting layer in the embodiment [2] are the same as those for the light-reflecting layer in the embodiment [1].

### [Adhesive Layer of Embodiment[2]]

The adhesive layer in the preferred embodiment [2] is an arbitrary layer formed so as to enhance the adhesion between the light-reflecting layer and the protective substrate.
The material constituting the adhesive layer is preferably photocurable resin, and for preventing warpage of the disc, more preferably a photocurable resin having a small curing shrinkage percentage. Examples of such a photocurable resin include a UV curable resin (UV curable adhesive) such as "SD-640" and "SD-347" produced by Dainippon Ink & Chemicals, Inc. The thickness of the adhesive layer is preferably from 1 to 1,000 µm so as to impart elasticity.

### [Protective Substrate of Embodiment [2]]

As for the protective substrate (dummy substrate) in the preferred embodiment [2], a substrate formed of the same construction material and having the same shape as the substrate described above may be used. The thickness of the protective substrate must be from 0.1 to 1.0 mm and is preferably from 0.2 to 0.8 mm, more preferably from 0.3 to 0.7 mm.

### [Protective Layer of Embodiment[2]]

In the optical information recording medium of the preferred embodiment [2], depending on the layer construction, a protective layer is provided for the purpose of physically and chemically protecting the light-reflecting layer, the write-once recording layer or the like.
Examples of the material used for the protective layer include an inorganic substance such as ZnS, ZnS-SiO₂, SiO, SiO₂, MgF₂, SnO₂ and Si₃N₄, and an inorganic substance such as thermoplastic resin, thermosetting resin and UV curable resin.
The protective layer can be formed, for example, by extruding a plastic and laminating the obtained film on the light-reflecting layer. The protective layer may also be provided by a method such as vacuum-deposition, sputtering and coating.

In the case of using a thermoplastic resin or a thermosetting resin, the protective layer may be formed by dissolving such a resin in an appropriate solvent to prepare a coating solution, and coating and drying the coating solution. In the case of a UV curable resin, the protective layer may be formed by using the resin as it is or by dissolving the resin in an appropriate solvent to prepare a coating solution, applying the coating solution, and curing the coating film by the irradiation with UV light. In the coating solution, various additives such as antistatic layer, antioxidant and UV absorbent may be further added according to the purpose.
The thickness of the protective layer is generally from 0.1 µm to 1 mm.

### [Other Layers in Embodiment [2]]

The optical information recording medium of the preferred embodiment [2] may have other arbitrary layers in addition to the above-described layers, within the range of not impairing the effects of the present invention. The detailed description of other arbitrary layers is the same as that for other layers in the embodiment [1].

### <Optical Information Recording Method>

The optical information recording method of the present invention is performed, for example, as follows by using the optical information recording medium of the preferred embodiment [1] or [2]. While rotating the optical information recording medium at a constant linear velocity (from 0.5 to 10 m/sec) or at a constant angular velocity, light for recording, such as semiconductor laser ray, is irradiated from the substrate side or the protective layer side. It is considered that upon this light irradiation, the recording layer absorbs the light and causes local elevation of the temperature to bring about physical or chemical changes (fox example, production of pits), whereby the optical properties are changed and the information is recorded. In the present invention, semiconductor laser light having an oscillation wavelength of 390 to 450 nm is used as the recording light. Preferred examples of the light source include a blue-violet semiconductor laser of emitting light having an oscillation wavelength of 390 to 415 nm, and a blue-violet SHG laser of emitting light having a center oscillation wavelength of 425 nm resulting from halving the wavelength of infrared semiconductor laser light having a center oscillation wavelength of 850 nm by using an optical waveguide device. In particular, a blue-violet semiconductor of emitting light having an oscillation wavelength of 390 to 415 nm is preferably used in view of the recording density. The thus-recorded information can be reproduced by irradiating semiconductor laser light from the substrate side or protective layer side while rotating the optical information recording medium at the same constant linear velocity as above, and detecting the reflected light.

### [Examples]

The present invention is described in greater detail below by referring to Examples, but the present invention is not limited to these Examples.

### Example 1:

[Synthesis of Compound (3)]

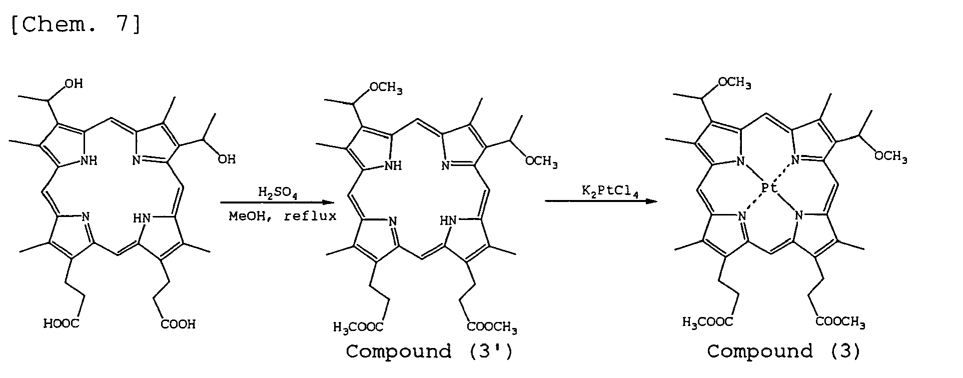

Hematoporphyrin (8 g) was dissolved in 80 ml of methanol, and 0.7 ml of sulfuric acid was added thereto, followed by refluxing under heat for 12 hours. The reaction solution was poured in 500 ml of water, thereby producing a precipitate, and the precipitate was filtered and then washed with water to obtain 6.1 g of Compound (3').
Subsequently, 4 g of Compound (3') and 7.6 g of K₂PtCl₄ were refluxed under heating for 8 hours in a mixed solvent containing 80 ml of methanol, 20 ml of water and 20 ml of toluene. The reaction solution was subjected to a liquid separation operation by adding ethyl acetate/water thereto, the organic layer was filtered through Celite, and then the solvent was removed by distillation. The resulting crude product was purified by silica gel chromatography (eluent: dichloromethane/methanol = 99/1) to obtain 360 mg of Compound (3). The compound obtained was identified by MALDI-MS. [M-H]⁺=848.

[Synthesis of Compound (B)]

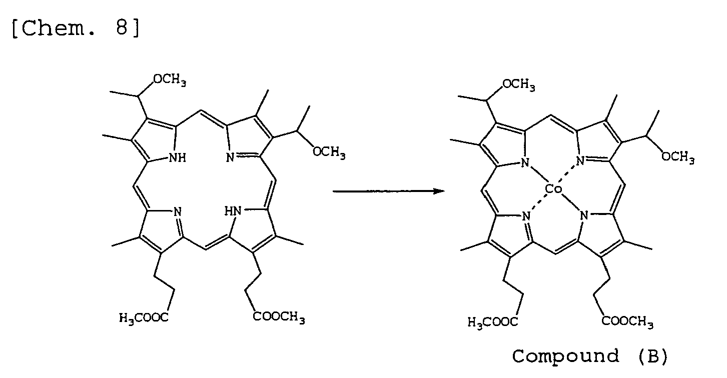

Compound (3') (0.7 g) and 0.27 g of Co(OAc)₂·4H₂O were refluxed under heating for 2 hours in a mixed solvent containing 7 ml of chloroform and 3 ml of methanol. The solvent was removed by distillation, and the resulting crude product was purified by silica gel chromatography (eluent: dichloromethane/methanol = 98/2) to obtain 420 mg of Compound (B). The obtained compound was identified by MALDI-TOFMS. [M-H]⁺=712.

[Synthesis of Compound (C)]

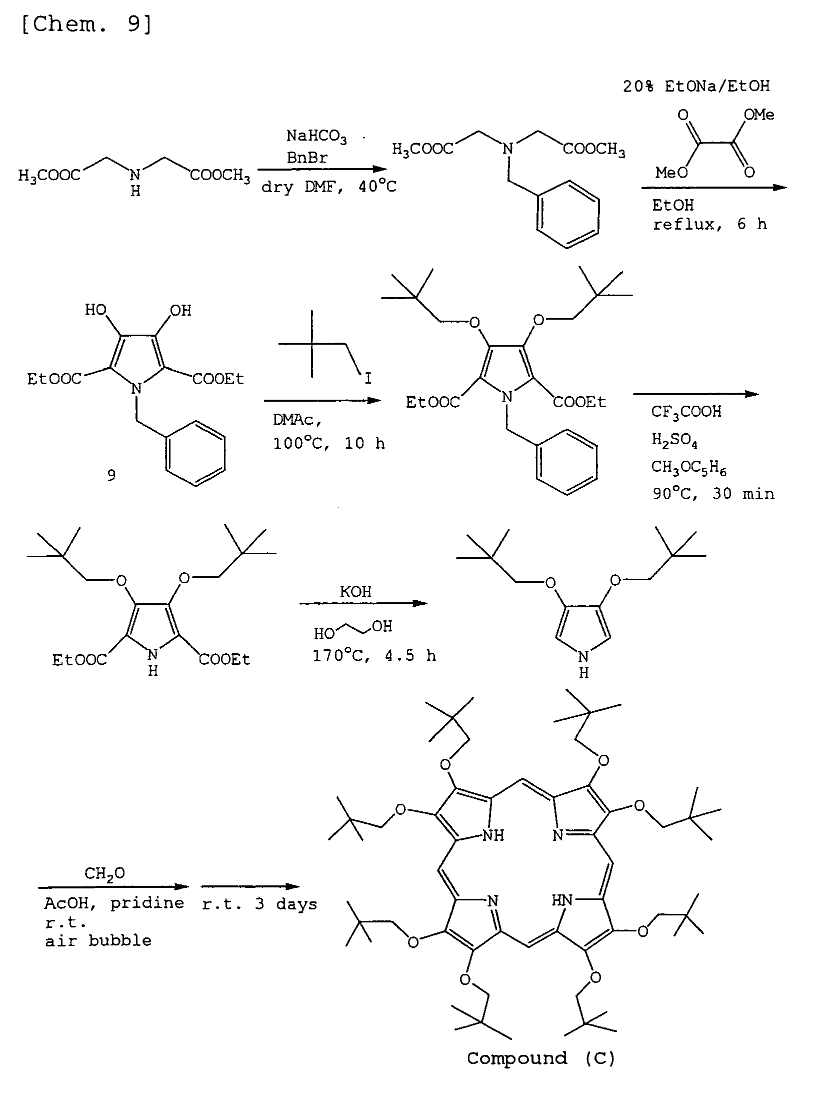

Compound (C) was synthesized according to the scheme shown above by referring to the method described in Synthesis, 795 (1995).
The obtained compound was identified by MALDI-TOFMS. [M-H]⁺=999.
Compound (12) is a compound described in the publication above and can be synthesized by the method described in the publication. Compound (7) can be synthesized in the same manner as Compound (3) by using Compound (12).

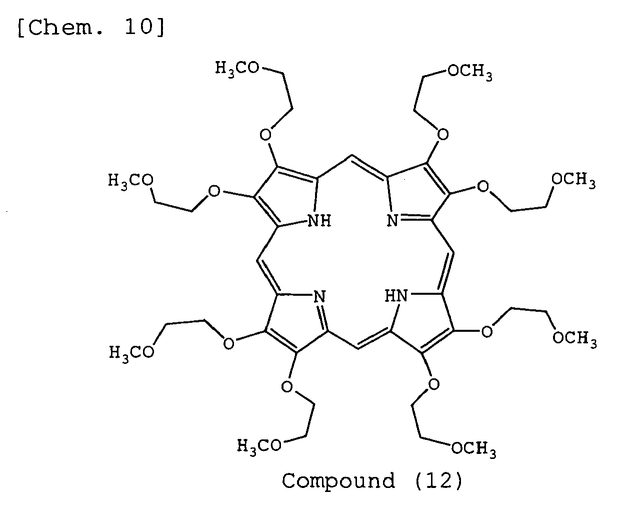

[Synthesis of Compound (D)]

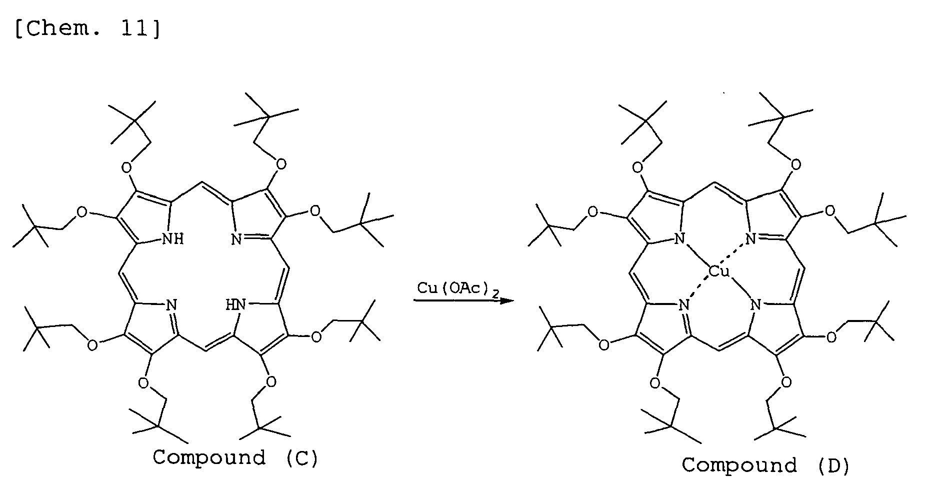

The obtained compound was identified by MALDI-TOFMS. [M-H]⁺=1060.

### Example 2:

### <Production of Optical Information Recording Medium 1>

### (Production of Substrate)

An injection molded substrate comprising a polycarbonate resin and having a thickness of 1.1 mm, an outer diameter of 120 mm, an inner diameter of 15 mm and a spiral groove (track pitch: 320 nm, groove width (on-groove width): 120 nm, groove depth: 35 nm, groove tilt angle: 65°, wobble amplitude: 20 nm) was produced. The mastering of the stamper used at the injection molding was performed by using laser cutting (351 nm).

### (Formation of Light-Reflecting Layer)

An APC light-reflecting layer (Ag: 98.1 mass%, Pd: 0.9 mass%, Cu: 1.0 mass%) as a vacuum-formed layer having a thickness of 100 nm was formed on the substrate in an Ar atmosphere by DC sputtering with use of Cube manufactured by Unaxis. The thickness of the light-reflecting layer was adjusted within the sputtering time.

### (Formation of Write-Once Recording layer)

The compound (0.2 g) shown in Table 1 was added and dissolved in 10 ml of 2,2,3,3-tetrafluoro-1-propanol to prepare a dye-containing coating solution. The dye-containing coating solution prepared was coated on the light-reflecting layer by spin coating while varying the rotation number from 300 to 4,000 rpm under the conditions of 23°C and 50% RH. Thereafter, the coating film was stored for 1 hour under the conditions of 23°C and 50% RH to form a write-once recording layer (thickness on groove: 120 nm, thickness on land: 170 nm). In the case of Compounds (D) and (E), a disc could not be produced, because these were sparingly soluble in a solvent.

After the write-once recording layer was formed, an annealing treatment as applied in a clean oven. In the annealing treatment, the substrate was supported by a vertical stack pole while taking space with a spacer and held at 80°C for 1 hour.

### (Formation of Barrier Layer)

A barrier layer comprising ZnO-Ga₂O₃ (ZnO:Ga₂O₃ = 7:3 (by mass)) and having a thickness of 5 nm was formed on the write-once recording layer in an Ar atmosphere by RF sputtering with use of Cube manufactured by Unaxis.

### (Lamination of cover Layer)

A polycarbonate-made film (Pure-Ace, produced by Teijin Ltd., thickness: 80 µm) having provided on one surface thereof a pressure-sensitive adhesive and having an inner diameter of 15 mm and an outer diameter of 120 mm was used for the cover layer, and the total thickness of the pressure-sensitive adhesive layer and the polycarbonate-made film was set to 100 µm.
The cover layer was placed on the barrier layer by abutting the barrier layer and the pressure-sensitive adhesive layer against each other and laminated by press-contacting the cover layer by means of a pressing member.
In this way, optical information recording mediums of Examples 1 and 2 and Comparative Examples 1 to 5 were produced.

The compound used in Comparative Example 1 is corresponding to Compound 34 described in JP-A-2001-138634.

### <Evaluation of Optical Information Recording Medium 1>

### (1) Evaluation of C/N (Carrier Wave/Noise Ratio)

Using a recording and reproduction evaluating apparatus (DDU1000, manufactured by Pulstec Industrial Co., Ltd.) having mounted thereon a 403-nm laser and a pickup of NA 0.85, the optical information recording medium produced was subjected to recording and reproduction of a 0.16 µm signal (2T) at a clock frequency of 66 MHz and a linear velocity of 5.28 m/s, and the reading of recording pits was performed. Incidentally, in this evaluation, the optical information recording method of the present invention was used, and the recording was made on grooves. The results are shown in Table 1.

**[Table 1]**

| Table 1 | | | | | | | |
|---|---|---|---|---|---|---|---|
| | Compound | Molar Extinction Coefficient (dm³mol⁻¹ cm⁻¹) | Solution Absorption Maximum Peak Wavelength [nm] | Film Absorption Refractive Maximum Peak Refractive Wavelength [nm] | Index, n | Extinction Coefficient, k | Reading of Recording Pits |
| Invention 1 | Compound (3) | 233000* | 377* | 380 | 2.1 | 0.4 | possible |
| Invention 2 | Compound (3) + Compound(7) | - | 377* | 380 | 2.0 | 0.4 | possible |
| Comparative Example 1 | Compound (E) | 259000** | 381** | 374*** | 2.3*** | 0.5*** | A disc could not be produced and evaluated due to sparing solubility in solvent. |
| Comparative Example 2 | Compound (B) | 159000* | 390* | 387 | 2.0 | 0.6 | impossible |
| Comparative Example 3 | Compound (A) | 110000* | 389* | 376 | 2.1 | 0.71 | impossible |
| Comparative Example 4 | Compound (D) | 190000* | 393** | 375**** | 2.0**** | 0.5**** | A disc could not be produced and evaluated due to sparing solubility in solvent. |

| | | | | | | | |
|---|---|---|---|---|---|---|---|
| * In 2,2,3,3-tetrafluoropropanol. ** In chloroform. *** Value as a vapor-deposition film. **** A coating film was produced by using 1,2-dichloroethane as the solvent. | | | | | | | |

As seen from the results in Table 1, samples using the compound of the present invention (Invention Examples 1 and 2) all are excellent in the recording and reproducing characteristics as compared with samples using the compounds of Comparative Examples 1 to 4.

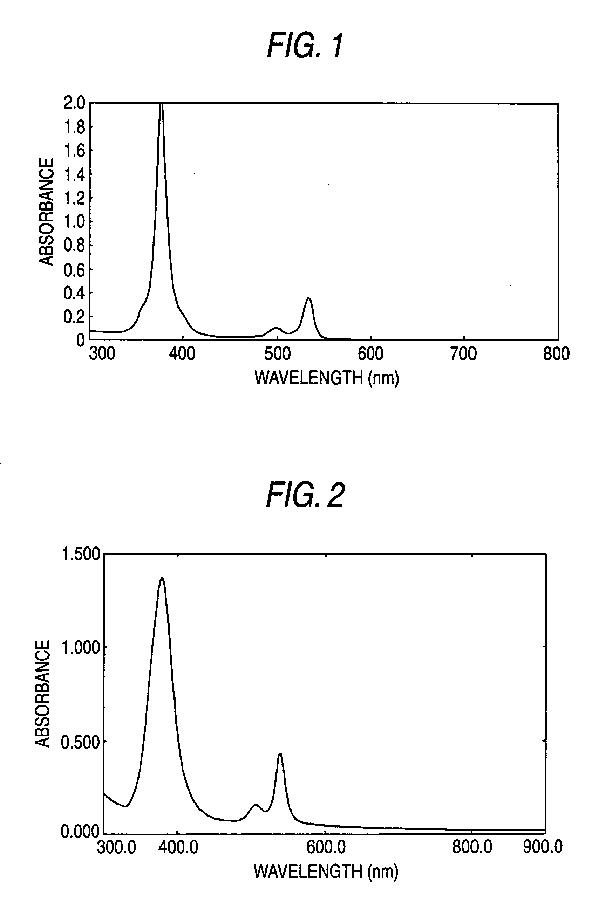

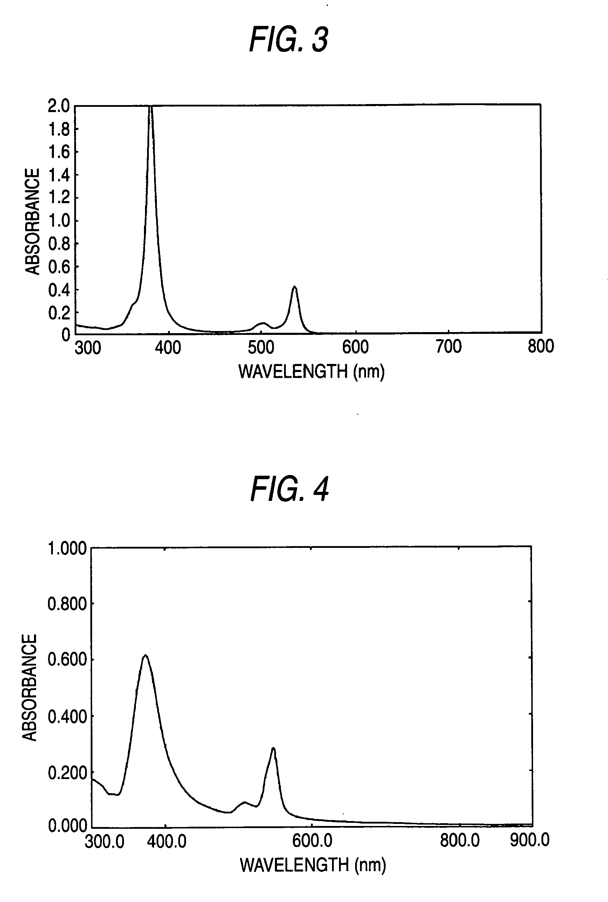

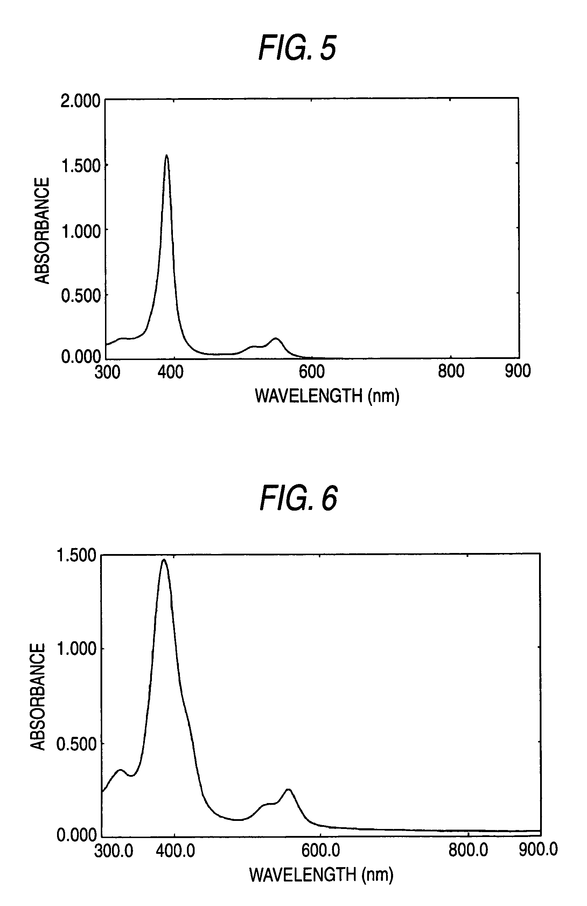

The solution absorption spectrum and the film absorption spectrum of each of the compounds obtained were measured. Figs. 1 to 6 show the results.

The solution absorption spectra of Compounds (G) to (L) (as for M, see Table 2) which are the compound within the category of JP-A-11-334207 were measured. The results are shown in Table 2. As seen from Table 2, the absorption maximum peak wavelengths of Compounds (G) to (L) are equal to the wavelength of Compound (B) or longer than the wavelength of Compound (B). Accordingly, Compounds (G) to (L) cannot satisfy n≥2.0 and 0.01≤k≤0.4 at the same time. These results reveal that when M=Pt, the absorption maximum peak becomes a peculiarly short wavelength as compared with other metals and the relationships of n≥2.0 and 0.01≤k≤0.4 can be satisfied at the same time.

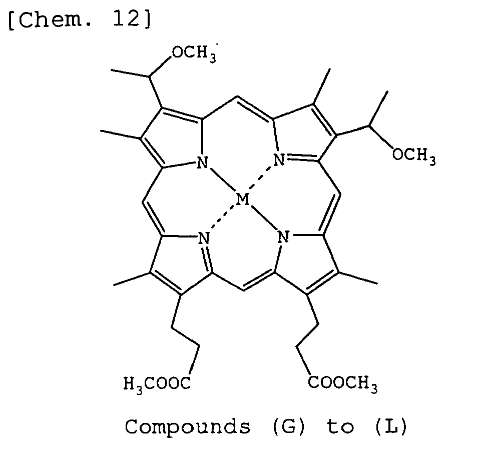

**[Table 2]**

| Table 2 | | |
|---|---|---|
| Compound No. | M | Solution Absorption Maximum Peak Wavelength [nm]* |
| Compound (G) | Fe | 400 |
| Compound (H) | Ni | 390 |
| Compound (I) | Zn | 403 |
| Compound (J) | Mg | 391 |
| Compound (K) | Cu | 394 |
| Compound (L) | H₂ | 395 |

| | | |
|---|---|---|
| * In 2,2,3,3-tetrafluoro-1-propanol. | | |

### INDUSTRIAL APPLICABILITY

The optical information recording medium of the present invention is useful as an optical information recording medium for use with a blue laser. The compound of the present invention is applicable to a pigment, a photographic material, a UV absorbing material, a laser dye, a color filter dye, a color conversion filter and the like, in addition to the optical information recording medium for use with a blue laser.

While the present invention has been described in detail and with reference to specific embodiments thereof, it will be apparent to one skilled in the art that various changes and modifications can be made therein without departing from the spirit and scope thereof.
This application is based on the Japanese patent application (Application No. 2005-075553) filed on March 16, 2005, the contents of which are incorporated by reference herein.

## Claims

1. An optical recording medium comprising a substrate having thereon a recording layer capable of recording information by the irradiation with laser light of 440 nm or less, wherein said recording layer comprises at least one platinum porphyrin represented by the following formula (I): 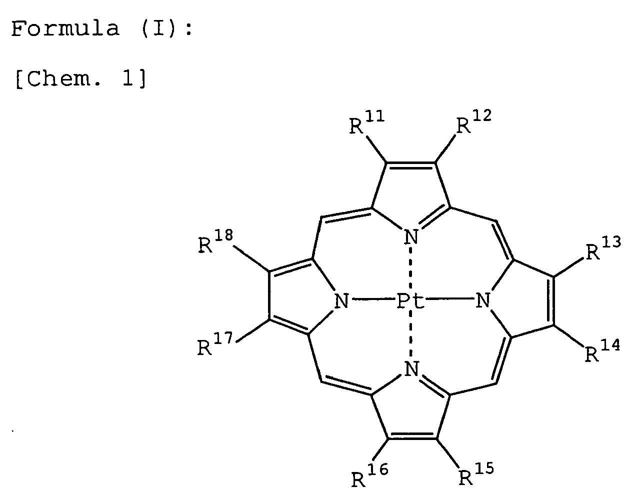 wherein R¹¹ to R¹⁸ each independently represents a hydrogen atom or a substituent.

2. The optical information recording medium as claimed in claim 1, wherein said recording layer comprises at least one platinum porphyrin represented by formula (I) and both the solution absorption maximum wavelength and the film absorption maximum wavelength of the platinum porphyrin represented by formula (I) are from 350 to 385 nm.

3. The optical information recording medium as claimed in claim 1 or 2, wherein said recording layer comprises at least one platinum porphyrin represented by formula (I) and the refractive index n and extinction coefficient k of said recording layer at the oscillation wavelength of laser light of 440 nm or less satisfy n≥2.0 and 0.01≤k≤0.4 at the same time.

4. The optical information recording medium as claimed in any one of claims 1 to 3, comprising a substrate having thereon a recording layer capable of recording information by the irradiation with laser light of 440 nm or less, wherein said recording layer comprises at least one platinum porphyrin represented by the following formula (II): 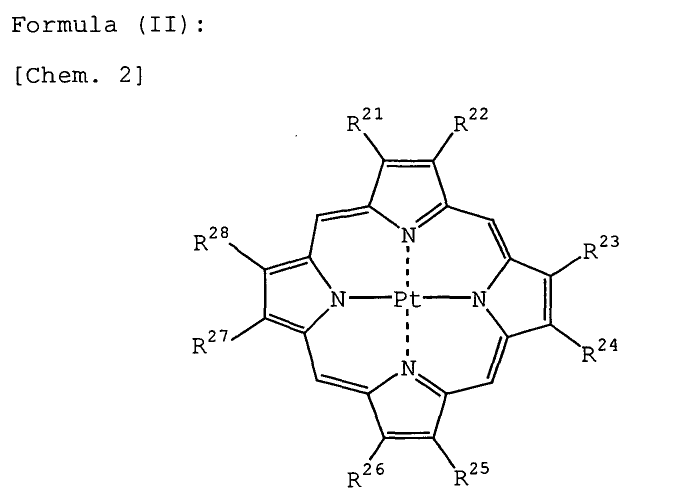 wherein R²¹ to R²⁸ each independently represents a substituted or unsubstituted alkyl group, provided that at least one substituent out of R²¹ to R²⁸ is a group further substituted by any one member of a hydroxyl group, a carboxyl group, an amino group, an alkylamino group, a mercapto group, an alkoxy group, an alkylthio group, an alkoxycarbonyl group, an alkylthiocarbonyl group, an alkylaminocarbonyl group and a heterocyclic group.

5. The optical information recording medium as claimed in any one of claims 1 to 4, wherein a light-reflecting layer comprising a metal is provided separately from said recording layer.

6. The optical information recording medium as claimed in any one of claims 1 to 5, wherein a protective layer is provided separately from said recording layer.

7. The optical information recording medium as claimed in any one of claims 1 to 6, wherein said substrate is a transparent disc-like substrate having on the surface thereof pregrooves at a track pitch of 0.2 to 0.5 mm and the recording layer is provided on said pregroove-formed surface.

8. The optical information recording medium as claimed in any one of claims 1 to 3, comprising a substrate having thereon a recording layer capable of recording information by the irradiation with laser light of 440 nm or less, wherein said recording layer comprises at least one platinum porphyrin represented by the following formula (III): 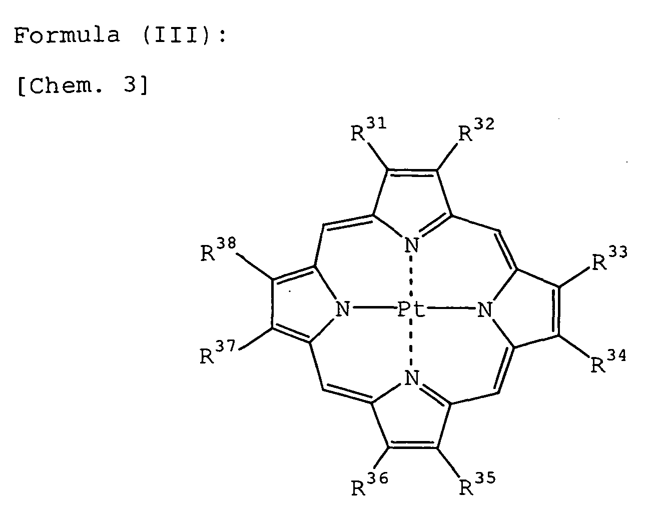 wherein R³¹ to R³⁸ each independently represents a substituted or unsubstituted alkoxy group or a substituted or unsubstituted alkylthio group, provided that at least one substituent out of R³¹ to R³⁸ is a group further substituted by any one member of a hydroxyl group, a carboxyl group, an amino group, an alkylamino group, a mercapto group, an alkoxy group, an alkylthio group, an alkoxycarbonyl group, an alkylthiocarbonyl group, an alkylaminocarbonyl group and a heterocyclic group.

9. The optical information recording medium as claimed in claim 8, wherein a light-reflecting layer comprising a metal is provided separately from said recording layer.

10. The optical information recording medium as claimed in claim 8 or 9, wherein a protective layer is provided separately from said recording layer.

11. The optical information recording medium as claimed in any one of claims 8 to 10, wherein said substrate is a transparent disc-like substrate having on the surface thereof pregrooves at a track pitch of 0.2 to 0.5 µm and the recording layer is provided on said pregroove-formed surface.
